# EUROPEAN PATENT APPLICATION

(11) **EP 1 978 017 A1**
(43) Date of publication of application: **08.10.2008**
(21) Application number: 06834132.0
(22) Date of filing: 06.12.2006
(51) Int. Cl.: C07D 211/94, C08F 2/40

(54) **NITROXYL COMPOUND, AND POLYMERIZATION INHIBITOR AND POLYMERIZATION INHIBITOR COMPOSITION USING SAME**

(30) Priority: 27.12.2005 JP 2005375673
(71) Applicant: Adeka Corporation, Tokyo 116-0012 (JP)
(72) Inventor: NEGISHI, Yoshinori, Saitama-shi, Saitama 3360022 (JP); TOBITA, Etsuo, Saitama-shi, Saitama 3360022 (JP); AYABE, Takashi, Saitama-shi, Saitama 3360022 (JP)
(74) Representative: Jones, Helen M.M.
(86) International application number: PCT/JP2006/324377
(87) International publication number: WO 2007/074613

(57) **Abstract**

The following compound, inhibitor, and composition are provided: a compound having low volatility, high nitroxyl group concentration, and high polymerization inhibitory activity; a polymerization inhibitor using the compound; and a polymerization inhibitor composition using the compound.

They are a nitroxyl compound represented by the following formula, a polymerization inhibitor using the nitroxyl compound, and a polymerization inhibitor composition using the nitroxyl compound:

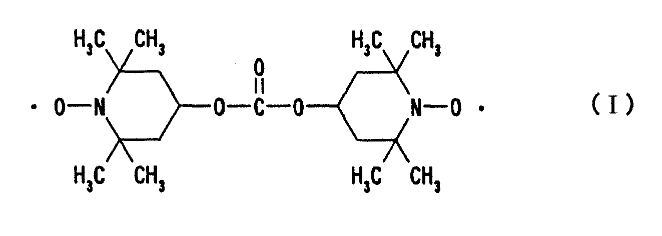

## Description

### Technical Field

The present invention relates to a nitroxyl compound, and a polymerization inhibitor and a polymerization inhibitor composition using the nitroxyl compound. The present invention particularly relates to a nitroxyl compound having low volatility and high polymerization inhibitory activity, a polymerization inhibitor and a polymerization inhibitor composition using the nitroxyl compound.

### Background Art

In general, hydroquinone, 1-oxy-2,2,6,6-tetramethyl-4-hydroxypiperidine, or the like is used to inhibit polymerization in a step of purifying or storing a radically polymerizable monomer such as an acrylic monomer or a styrene monomer.

In particular, 1-oxy-2,2,6,6-tetramethyl-4-hydroxypiperidine is used as a polymerization inhibitor and has high advantages as disclosed in Patent Documents 1 and 2.

Furthermore, 1-oxy-2,2,6,6-tetramethyl-4-hydroxypiperidine is known to impart high weather resistance to synthetic resins or the like.
Patent Document 1: Japanese Unexamined Patent Application Publication No. 5-320205 (claims and the like)
Patent Document 2: PCT Japanese Translation Patent Publication No. 10-504317 (claims and the like)

### Disclosure of Invention

### Problems to be Solved by the Invention

If 1-oxy-2,2,6,6-tetramethyl-4-hydroxypiperidine is heated to purify by distillation a monomer, however, 1-oxy-2,2,6,6-tetramethyl-4-hydroxypiperidine has high volatility and therefore may cause contamination of the purified monomer and polymerization because of dissipation of 1-oxy-2,2,6,6-tetramethyl-4-hydroxypiperidine due to its volatility. If 1-oxy-2,2,6,6-tetramethyl-4-hydroxypiperidine is used as a stabilizer for synthetic resins, 1-oxy-2,2,6,6-tetramethyl-4-hydroxypiperidine dissipates and therefore stabilizing effects are reduced with time. On the other hand, bis(1-oxyl-2,2,6,6-tetramethylpiperidine-4-yl) sebacate, which is disclosed in Patent Document 2, has a large molecular weight and therefore has a low polymerization-inhibiting effect per unit content because of the low concentration of a nitroxyl group therein.

Accordingly, it is an object of the present invention to provide a nitroxyl compound which has low volatility, a high nitroxyl group concentration, and high polymerization inhibitory activity, and a polymerization inhibitor and a polymerization inhibitor composition using the nitroxyl compound.

### Means for Solving the Problems

The inventors have made intensive investigations to solve the above problems and have found that a nitroxyl compound having a structure obtained by dimerizing 1-oxy-2,2,6,6-tetramethyl-4-hydroxypiperidine with a carbonate has low volatility and polymerization inhibitory activity close to that of 1-oxy-2,2,6,6-tetramethyl-4-hydroxypiperidine. This has resulted in the completion of the present invention.

A nitroxyl compound according to the present invention is represented by the following formula:

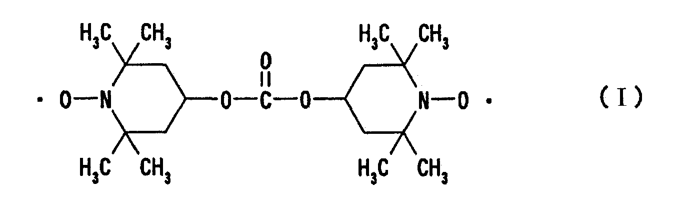

A polymerization inhibitor according to the present invention contains the nitroxyl compound according to the present invention.

A polymerization inhibitor composition according to the present invention contains the nitroxyl compound according to the present invention.

### Advantages

According to the present invention, the following compound, inhibitor, and composition can be provided: a nitroxyl compound having low volatility and high polymerization inhibitory activity, and a polymerization inhibitor and a polymerization inhibitor composition using the nitroxyl compound.

### Best Modes for Carrying Out the Invention

Hereinafter, the present invention will be described in detail.

A nitroxyl compound according to the present invention is bis(1-oxy-2,2,6,6-tetramethylpiperidine-4-yl) carbonate represented by the following formula and is referred to as a hindered amine compound: 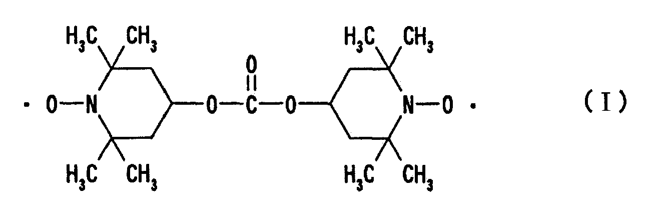 A method for producing the nitroxyl compound is not particularly limited. The nitroxyl compound can be produced by an ordinary method. The nitroxyl compound can be produced in such a manner that, for example, 4-hydroxy-l-oxy-2,2,6,6-tetramethylpiperidine is allowed to react with dimethyl carbonate or the like in a solvent in the presence of a base.

A monomer of which the polymerization is inhibited by the above-described nitroxyl compound, which is represented by Formula (I), is radically polymerizable and is not particularly limited. Examples of the monomer include unsaturated carboxylic acids such as acrylic acid, methacrylic acid, itaconic acid, crotonic acid, maleic acid, and fumaric acid; esters of the unsaturated carboxylic acids and mono- or poly-alcohols (for example, methanol, ethanol, butanol, allyl alcohol, octanol, ethylene glycol, diethylene glycol, triethylene glycol, propylene glycol, neopentyl glycol, 1,4-butane diol, 1,3-butane diol, 1,6-hexane diol, glycerin, trimethylol propane, trimethylol ethane, tris(2-hydroxyethyl) isocyanate, pentaerythritol, diglycerin, ditrimethylolpropane, and dipentaerythritol); amides of the unsaturated carboxylic acids and mono- or poly-amines (for example, ammonia, methyl amine, octyl amine, diethyl amine, dibutyl amine, ethylene diamine, diethylene triamine, piperazine, 1,6-hexamethylene diamine, and melamine); (meth)acrylates of polyhydroxy esters obtained from polycarboxylic acids and poly-alcohols; unsaturated polyesters obtained from unsaturated dicarboxylic acids (for example, maleic acid, provided that other acids (for example, phthalic acid, isophthalic acid, terephthalic acid, trimellitic acid, pyromellitic acid, tetrahydrophthalic acid, succinic acid, adipic acid, and sebacic acid) can be contained) and poly-alcohols; unsaturated polyamides obtained from these unsaturated dicarboxylic acids and poly-amines; urethane acrylates obtained from the reaction of hydroxyalkyl (meth)acrylates with urethane compounds, obtained from diisocyanates (for example, toluene diisocyanate, diphenylmethane diisocyanate, isocyanatomethyl cyclohexane isocyanate, hexamethylene diisocyanate, and isophorone diisocyanate) and poly-alcohols, having an end isocyanate group; epoxy acrylates obtained from the reaction of (meth)acrylates with polyglycidyl ethers of bis-phenols or polyols; linear polyesters obtained from the reaction of dicarboxylic anhydrides with glycidyl (meth)acrylates; di(meth)acryl-modified polyesters obtained from the reaction of glycidyl (meth)acrylates and polyesters having carboxylic groups at both ends; di(meth)acryl-modified polyamides obtained from the reaction of glycidyl (meth)acrylates and polyamides having carboxylic groups at both ends; vinyl compounds; and acrylonitrile.

Examples of the vinyl compounds include methyl acrylate, ethyl acrylate, butyl acrylate, isooctyl acrylate, 2-hydroxyethyl acrylate, methyl methacrylate, ethyl methacrylate, ethylene glycol diacrylate, triethylene glycol diacrylate, 1,4-butanediol diacrylate, pentaerythritol triacrylate, pentaerythritol tetraacrylate, tris(2-hydroxyethyl) isocyanurate triacrylate, dipentaerythritol tetra- to hexa-acrylates (alone or mixture of dipentaerythritol tetraacrylate, dipentaerythritol pentaacrylate, and dipentaerythritol hexaacrylate), glycerin diacrylate, triethylene glycol dimethacrylate, trimethylol propane trimethacrylate, pentaerythritol trimethacrylate, ethylene glycol dicronate, diallyl maleate, bis(acryloyloxyethylphenyl) propane, acrylamide, methacrylamide, ethylene bisacrylamide, hexamethylene bisacrylamide, methylene bismethacrylamide, bis(2-acryloyloxyethyl) hexamethylene dicarbamate, bis(2-hydroxy-3-acryloyloxypropyl) hexamethylene dicarbamate, 6-isocyanatohexane carbamoyloxyethyl acrylate, 6-isocyanatohexane carbamoyloxyethyl methacrylate, diallyl phthalate, diallyl maleate, divinyl adipate, divinyl phthalate, vinyl acetate, isobutyl vinyl ether, ethylene glycol divinyl ether, styrene, acrylonitrile, triallyl isocyanurate, triallyl phosphate, acrolein, methacrolein, butadiene, divinylbenzene, α-methylstyrene, vinyl pyridine, vinyl chloride, styrene, α-methylstyrene, vinyltoluene, divinylbenzene, styrenesulfonic acid, and acrylonitrile.

A polymerization inhibitor composition according to the present invention can be added to a vinyl compound in a step of producing, purifying, storing, or transporting the vinyl compound. The amount of the polymerization inhibitor composition added is preferably 0.001% to 3% by weight of the vinyl compound. The polymerization inhibitor composition has a function of scavenging radicals generated by heating the vinyl compound.

When the nitroxyl compound, which is represented by Formula (I) according to the present invention, is used as a polymerization inhibitor, the nitroxyl compound may be used in combination with another polymerization inhibitor. Examples of this polymerization inhibitor include phenolic polymerization inhibitors such as hydroquinone, p-methoxy phenol, and catechol; phenothiazinic polymerization inhibitors such as phenothiazine, bis (α-methylbenzyl) phenothiazine, 3,7-dioctylphenothiazine, and bis(α,α-dimethylbenzyl) phenothiazine; metal salt polymerization inhibitors such as copper or manganese salts of dimethyldithiocarbamic acid, diethyldithiocarbamic acid, dipropyldithiocarbamic acid, dibutyldithiocarbamic acid, diphenyldithiocarbamic acid, formic acid, acetic acid, octanoic acid, naphthenic acid, and ethylenediaminetetraacetic acid; and nitroso polymerization inhibitors such as p-nitrosophenol, N-nitrosodiphenylamine, and the ammonium salt (cupferron) of N-nitrosophenylhydroxyamine.

The nitroxyl compound, which is represented by Formula (I), is also useful as a stabilizer for synthetic resins. Examples of a synthetic resin stabilized therewith include α-olefin polymers or copolymers such as polypropylenes, low-density polyethylenes, linear low-density polyethylenes, high-density polyethylenes, polybutene-1, poly-3-methylpentene, poly-4-methylpentene, and ethylene-propylene copolymers; copolymers of α-olefins and acrylic acid, methacrylic acid, vinyl acetate, or poly-unsaturated compounds such as conjugated dienes and non-conjugated dienes; linear or acid-modified polyesters such as polyethylene terephthalate, polyethylene terephthalate-isophthalate, polyethylene terephthalate-paraoxybenzoate, and polybutylene terephthalate; polyamides such as polycaprolactam and polyhexamethylene adipamide; polyimides; polystyrenes; copolymers (for example, AS resins, ABS resins, MBS resins, and heat-resistant ABS resins) of styrene, a monomer (for example, maleic anhydride, phenylmaleimide, methyl methacrylate, butadiene, and acrylonitrile), and/or α-methyl styrene; halogen-containing resins such as polyvinyl chlorides, polyvinylidene chlorides, chlorinated polyethylenes, chlorinated polypropylenes, polyvinylidene fluorides, chlorinated rubbers, vinyl chloride-vinyl acetate copolymers, vinyl chloride-ethylene copolymers, vinyl chloride-vinylidene chloride copolymers, vinyl chloride-vinylidene chloride-vinyl acetate terpolymers, vinyl chloride-acrylate copolymers, vinyl chloride-maleate copolymers, and vinyl chloride-cyclohexylmaleimide copolymers; polymers of (meth)acrylates such as methyl (meth)acrylate, ethyl (meth)acrylate, and octyl (meth)acrylate; thermoplastic resins such as polyether ketones, polyvinyl acetates, polyvinyl formals, polyvinyl butyrals, polyvinyl alcohols, linear or branched polycarbonates, petroleum resins, coumarone resins, polyphenylene oxides, polyphenylene sulfides, polyurethanes, and cellulose resins; thermosetting resins such as epoxy resins, phenol resins, urea resins, melamine resins, and unsaturated polyesters; isoprene rubbers; butadiene rubbers; butadiene-styrene copolymer rubbers; butadiene-acrylonitrile copolymer rubbers; acrylonitrile-butadiene-styrene copolymer rubbers; copolymer rubbers of ethylene and α-olefins such as propylene and butene-1; elastomers such as ternary copolymer rubbers of ethylene-α-olefins, and non-conjugated dienes such as ethylidene norbornene and cyclopentadiene; and silicones. These resins and/or elastomers may be alloyed or blended.

Although the stabilization of the synthetic resin depends on the tacticity thereof, the density thereof, the type of a polymerization catalyst used, the presence or removal of the polymerization catalyst, the degree of crystallization, polymerization conditions such as temperature and pressure, the type of a crystal, the size of a crystal lamella analyzed by small-angle X-ray scattering, the aspect ratio of crystal grains, the solubility in an aromatic or aliphatic solvent, the solution viscosity thereof, the melt viscosity thereof, the average molecular weight thereof, the molecular weight distribution thereof, the number of peaks in the molecular weight distribution, whether the synthetic resin is a block or random copolymer, and the ratio of monomers used, the nitroxyl compound can be used for any resin.

When the nitroxyl compound, which is represented by Formula (I) according to the present invention, is used for the stabilization of the synthetic resin, various types of agents may be used as required in combination with the nitroxyl compound. Examples of the agents include phenolic antioxidants, sulfuric antioxidants, phosphoric antioxidants, ultraviolet absorbers, other hindered amine compounds, nucleating agents, flame retardants, flame retardant aids, lubricants, fillers, fibrous fillers, metal soaps, hydrotalcites, antistatic agents, pigments, and dyes.

Examples of the phenolic antioxidants include 2,6-di-tert-butyl-p-cresol, 2,6-diphenyl-4-octadecyloxyphenol, distearyl(3,5-di-tert-butyl-4-hydroxybenzyl) phosphonate, 1,6-hexamethylenebis[(3,5-di-tert-butyl-4-hydroxyphenyl) propioamide], 4,4'-thiobis(6-tert-butyl-m-cresol), 2,2'-methylenebis(4-methyl-6-tert-butylphenol), 2,2'-methylenebis(4-ethyl-6-tert-butylphenol), 4,4'-butylidenebis(6-tert-butyl-m-cresol), 2,2'-ethylidenebis(4,6-di-tert-butylphenol), 2,2'-ethylidenebis(4-sec-butyl-6-tert-butylphenol), 1,1,3-tris(2-methyl-4-hydroxy-5-tert-butylphenyl)butane, 1,3,5-tris(2,6-dimethyl-3-hydroxy-4-tert-butylbenzyl) isocyanurate, 1,3,5-tris(3,5-di-tert-butyl-4-hydroxybenzyl) isocyanurate, 1,3,5-tris(3,5-di-tert-butyl-4-hydroxybenzyl)-2,4,6-trimethylbenzene, 2-tert-butyl-4-methyl-6-(2-acryloyloxy-3-tert-butyl-5-methylbenzyl)phenol, stearyl(3,5-di-tert-butyl-4-hydroxyphenyl) propionate, thiodietylene glycol bis[(3,5-di-tert-butyl-4-hydroxyphenyl) propionate], 1,6-hexamethylenebis[(3,5-di-tert-butyl-4-hydroxyphenyl) propionate], bis[3,3-bis(4-hydroxy-3-tert-butylphenyl)butyric acid]glycol ester, bis[2-tert-butyl-4-methyl-6-(2-hydroxy-3-tert-butyl-5-methylbenzyl)phenyl] terephthalate, 1,3,5-tris[(3,5-di-tert-butyl-4-hydroxyphenyl)propionyloxyethyl] isocyanurate, 3,9-bis[1,1-dimethyl-2-{(3-tert-butyl-4-hydroxy-5-methylphenyl)propionyloxy}ethyl]-2,4,8,10-tetraoxaspiro[5.5]undecane, and triethylene glycol bis[(3-tert-butyl-4-hydroxy-5-methylphenyl) propionate]. The amount of each phenolic antioxidant added is preferably 0.001 to 10 parts and more preferably 0.05 to 5 parts per 100 parts of a resin on a weight basis.

Examples of the sulfuric antioxidants include dialkyl thiodipropionates such as dilauryl thiodipropionate, dimyristyl thiodipropionate, and distearyl thiodipropionate and polyol β-alkylmercaptopropionates such as pentaerythritol tetra(β-dodecylmercaptopropionate). The amount of each sulfuric antioxidant added is preferably 0.001 to 10 parts and more preferably 0.05 to 5 parts per 100 parts of a resin on a weight basis.

Examples of the phosphoric antioxidants include trisnonylphenyl phosphite, tris[2-tert-butyl-4-(3-tert-butyl-4-hydroxy-5-methylphenylthio)-5-methylphenyl] phosphite, tridecyl phosphite, octyl diphenyl phosphite, di(decyl) monophenyl phosphite, di(tridecyl) pentaerythritol diphosphite, di(nonylphenyl) pentaerythritol diphosphite, bis(2,4-di-tert-butylphenyl) pentaerythritol diphosphite, bis(2,6-di-tert-butyl-4-methylphenyl) pentaerythritol diphosphite, bis(2,4,6-tri-tert-butylphenyl) pentaerythritol diphosphite, bis(2,4-dicumylphenyl) pentaerythritol diphosphite, tetra(tridecyl)isopropylidenediphenol diphosphite, tetra(tridecyl)-4,4'-n-butylidenebis(2-tert-butyl-5-metylphenol) diphosphite, hexa(tridecyl)-1,1,3-tris(2-methyl-4-hydroxy-5-tert-butylphenyl)butane triphosphite, tetrakis(2,4-di-tert-butylphenyl)biphenylene diphosphonite, 9,10-dihydro-9-oxa-10-phosphaphenanthrene-10-oxide, 2,2'-methylenebis(4,6-tert-butylphenyl)-2-ethylhexyl phosphite, 2,2'-methylenebis(4,6-tert-butylphenyl)-octadecyl phosphite, 2,2'-ethylidenebis(4,6-di-tert-butylphenyl) fluorophosphite, tris(2-[(2,4,8,10-tetrakis-tert-butyldibenzo[d,f][1,3,2]dioxaphosphepin-6-yl)oxy]ethyl)amine, and a phosphite of 2-ethyl-2-butylpropylene glycol and 2,4,6-tri-tert-butylphenol. The amount of each phosphoric antioxidant added is preferably 0.001 to 10 parts and more preferably 0.05 to 5 parts per 100 parts of a resin on a weight basis.

Examples of the ultraviolet absorbers include 2-hydroxybenzophenones such as 2,4-dihydroxybenzophenone, 2-hydroxy-4-methoxybenzophenone, 2-hydroxy-4-octoxybenzophenone, and 5,5'-methylenebis(2-hydroxy-4-methoxybenzophenone); 2-(2'-hydroxyphenyl)benzotriazoles such as 2-(2'-hydroxy-5'-methylphenyl)benzotriazole, 2-(2'-hydroxy-3',5'-di-tert-butylphenyl)-5-chlorobenzotriazole, 2-(2'-hydroxy-3'-tert-butyl-5'-methylphenyl)-5-chlorobenzotriazole, 2-(2'-hydroxy-5'-tert-octylphenyl)benzotriazole, 2-(2'-hydroxy-3',5'-dicumylphenyl)benzotriazole, and 2-(2'-hydroxy-3'-tert-butyl-5'-carboxyphenyl)benzotriazole; benzoates such as phenyl salicylate, resorcinol monobenzoate, 2,4-di-tert-butylphenyl-3,5-di-tert-butyl-4-hydroxybenzoate, 2,4-di-tert-amylphenyl-3,5-di-tert-butyl-4-hydroxybenzoate, and hexadecyl-3,5-di-tert-butyl-4-hydroxybenzoate; substituted oxanilides such as 2-ethyl-2'-ethoxyoxanilide and 2-ethoxy-4'-dodecyloxanilide; cyanoacrylates such as ethyl-α-cyano-β,β-diphenylacrylate and methyl-2-cyano-3-methyl-3-(p-methoxyphenyl)acrylate; and triaryltriazines such as 2-(2-hydroxy-4-octoxyphenyl)-4,6-bis(2,4-di-tert-butylphenyl)-s-triazine, 2-(2-hydroxy-4-methoxyphenyl)-4,6-diphenyl-s-triazine, and 2-(2-hydroxy-4-propoxy-5-methylphenyl)-4,6-bis(2,4-di-tert-butylphenyl)-s-triazine. The amount of each ultraviolet absorber added is preferably 0.001 to 10 parts and more preferably 0.05 to 5 parts per 100 parts of a resin on a weight basis.

Examples of the above-described other hindered amine compounds include 2,2,6,6-tetramethyl-4-piperidyl stearate, 1,2,2,6,6-pentamethyl-4-piperidyl stearate, 2,2,6,6-tetramethyl-4-piperidyl benzoate, bis(2,2,6,6-tetramethyl-4-piperidyl) sebacate, bis(1,2,2,6,6-pentamethyl-4-piperidyl) sebacate, tetrakis(2,2,6,6-tetramethyl-4-piperidyl)butane tetracarboxylate, tetrakis(1,2,2,6,6-pentamethyl-4-piperidyl)butane tetracarboxylate, bis(2,2,6,6-tetramethyl-4-piperidyl)di(tridecyl)-1,2,3,4-butane tetracarboxylate, bis(1,2,2,6,6-pentamethyl-4-piperidyl)di(tridecyl)-1,2,3,4-butane tetracarboxylate, bis(1,2,2,6,6-pentamethyl-4-piperidyl)-2-butyl-2-(3,5-di-tert-butyl-4-hydroxybenzyl) malonate, polycondensates of 1-(2-hydroxyethyl)-2,2,6,6-tetramethyl-4-piperidinol and diethyl succinate, polycondensates of 1,6-bis(2,2,6,6-tetramethyl-4-piperidylamino)hexane and dibromoethane, polycondensates of 1,6-bis(2,2,6,6-tetramethyl-4-piperidylamino)hexane and 2,4-dichloro-6-morpholino-s-triazine, polycondensates of 1,6-bis(2,2,6,6-tetramethyl-4-piperidylamino)hexane and 2,4-dichloro-6-tert-octylamino-s-triazine, 1,5,8,12-tetrakis[2,4-bis(N-butyl-N-(2,2,6,6-tetramethyl-4-piperidyl)amino)-s-triazin-6-yl]-1,5,8,12-tetrazadodecane, 1,5,8,12-tetrakis[2,4-bis(N-butyl-N-(1,2,2,6,6-pentamethyl-4-piperidyl)amino)-s-triazin-6-yl]-1,5,8,12-tetrazadodecane, 1,6,11-tris[2,4-bis(N-butyl-N-(2,2,6,6-tetramethyl-4-piperidyl)amino)-s-triazin-6-yl]aminoundecane, and 1,6,11-tris[2,4-bis(N-butyl-N-(1,2,2,6,6-pentamethyl-4-piperidyl)amino)-s-triazin-6-yl]aminoundecane.

Examples of the nucleating agents include metal aromatic carboxylates such as aluminum p-tert-butylbenzoate and sodium benzoate; acidic metal phosphates such as sodium bis(2,4-di-tert-butylphenyl)phosphate, lithium bis(2,4-di-tert-butylphenyl)phosphate, and sodium 2,2'-methylenebis(4,6-di-tert-butylphenyl)phosphate; and polyalcohol derivatives such as dibenzylidene sorbitol and bis(methylbenzylidene) sorbitol.

Examples of the flame retardants include halogen flame retardants, red phosphorus, melamine phosphate, guanidine phosphate, phosphoric acid esters, phosphorus flame retardants such as phosphazene compounds, nitrogen flame retardants such as melamine cyanurate, and metal hydroxides such as magnesium hydroxide and aluminum hydroxide. Examples of the flame retardant aids include inorganic compounds such as antimony trioxide and zinc borate and anti-drip agents such as polytetrafluoroethylene.

The hydrotalcites may be natural or synthetic and can be used independently of surface treatment or the presence of water of crystallization. Examples of the hydrotalcites include basic carbonates represented by the following formula (II):

MₓMg_{y}Al_{z}CO₃(OH)_{xp+2y+3z-2}·nH₂O (II)

wherein M represents an alkali metal or zinc, x represents a number from 0 to 6, y represents a number from 0 to 6, z represents a number from 0.1 to 4, p represents the valence of M, and n represents the number of water of crystallization molecules and ranges from 0 to 100.

Examples of the lubricants include fatty acid amides such as laurylamide, myristylamide, laurylamide, stearylamide, and behenylamide; ethylenebisstearylamide; polyethylene wax; metal soaps such as calcium stearate and magnesium stearate; and metal phosphates such as magnesium distearyl phosphate and magnesium stearyl phosphate.

Examples of the fillers include inorganic compounds such as talc, silica, calcium carbonate, fibrous glass, potassium titanate, and potassium borate. When the fillers are particulate, the fillers have an appropriately chosen particle size. When the fillers are fibrous, the fillers have an appropriately chosen diameter, length, and aspect ratio. The fillers are preferably surface-treated as required.

For agricultural film uses, an ultraviolet absorber can be used to control the growth of the plants, an infrared absorber can be used to improve heat insulation, and also anti-fogging additive and anti-mist agent can be used because plants may receive an insufficient amount of light due to fog in vinyl houses and dewfall on films.

The nitroxyl compound according to the present invention is preferably used for the stabilization of synthetic resins, particularly for the stabilization of polyolefin agricultural films, and may be used for the long-term stabilization of organic matter, for example, the stabilization of liquids such as lubricants and electrolytic solutions.

### Examples

The present invention will now be further described in detail with reference to examples. The present invention is not limited to the examples.

### Synthesis Example 1

The following materials were charged into a 300 mL four-neck flask: 40.0 g (0.231 mol) of 1-oxy-2,2,6,6-tetramethyl-4-hydroxypiperidine, 1.5 g (7.9 mmol) of a 28% methanol solution of sodium methoxide, 20.8 g (0.231 mol) of dimethyl carbonate, and mineral spirit (21.4 g). Methanol was distilled off from the mixture at 80°C for two hours. The resulting mixture was heated to 100°C over two hours and then maintained for one hour. The reaction mixture was analyzed by gas chromatography (the system GC-14B available from Shimadzu Corporation; a 1 m long, 2.6 mm diameter column, packed with 5% silicon OV-17 on Chromosorb WAW (60-80 mesh), available from GL Science Inc.; an injection temperature of 250°C; a detector temperature of 300°C; and a heating rate of 15°C/minute). This showed that the conversion was 93%. The solvent was distilled off from the reaction mixture under a reduced pressure at 100°C. The resulting reaction mixture was cooled and then dissolved in 200 mL of ethyl acetate. The ethyl acetate solution was neutralized with a 1 mol/L aqueous solution of hydrochloric acid. The aqueous layer was separated from the organic layer, which was then washed with 30 mL of water. The organic layer was dried with magnesium sulfate. A solid was removed from the organic layer by filtration. The filtrate was concentrated in an evaporator (80°C, 10 mmHg or less), whereby a red crystal with a melting point of 195.3°C was obtained.

The analysis results of the obtained compound were as shown below.
Infrared absorption spectrum (KBr, cm⁻¹): 2950, 2920, 1750, 1320, 1275, 1240, 1180, and 960
MASS (GC-MS, m/z): 371, 355, 340, 202, 154, 140, 124, 109, 100, 82, and 74

### Examples 1-1 to 1-3 and Comparative Examples 1-1 to 1-5

Properties of each polymerization inhibitor were evaluated by the following procedure: 3 g of methacrylic acid was dissolved in 30 g of toluene, azoisobutylonitrile (hereinafter referred to as "AIBN") and the polymerization inhibitor were added to the solution, and the time taken for the resulting solution to become cloudy at 90°C was then measured. The polymerization inhibitor was subjected to thermal analysis (the analyzer Thermoplus TG8120 available from Rigaku Corporation, a heating rate of 10°C/minute, and an air flow rate of 200 ml/s) and was evaluated for volatility using the 5% weight loss temperature thereof.
The amount of AIBN added, the type and amount of the polymerization inhibitor added, and obtained results were summarized in Table 1. The polymerization inhibitor used in Examples 1-1 to 1-3 was the nitroxyl compound (referred to as "Formula (I)" in tables below), represented by Formula I, according to the present invention. No polymerization inhibitor was used in Comparative Example 2-1, 2-3, or 2-5. The polymerization inhibitor used in Comparative Example 2-1, 2-3, or 2-5 was 1-oxy-2,2,6,6-tetramethyl-4-hydroxypiperidine (referred to as "Comparative Compound 1" in the below tables).

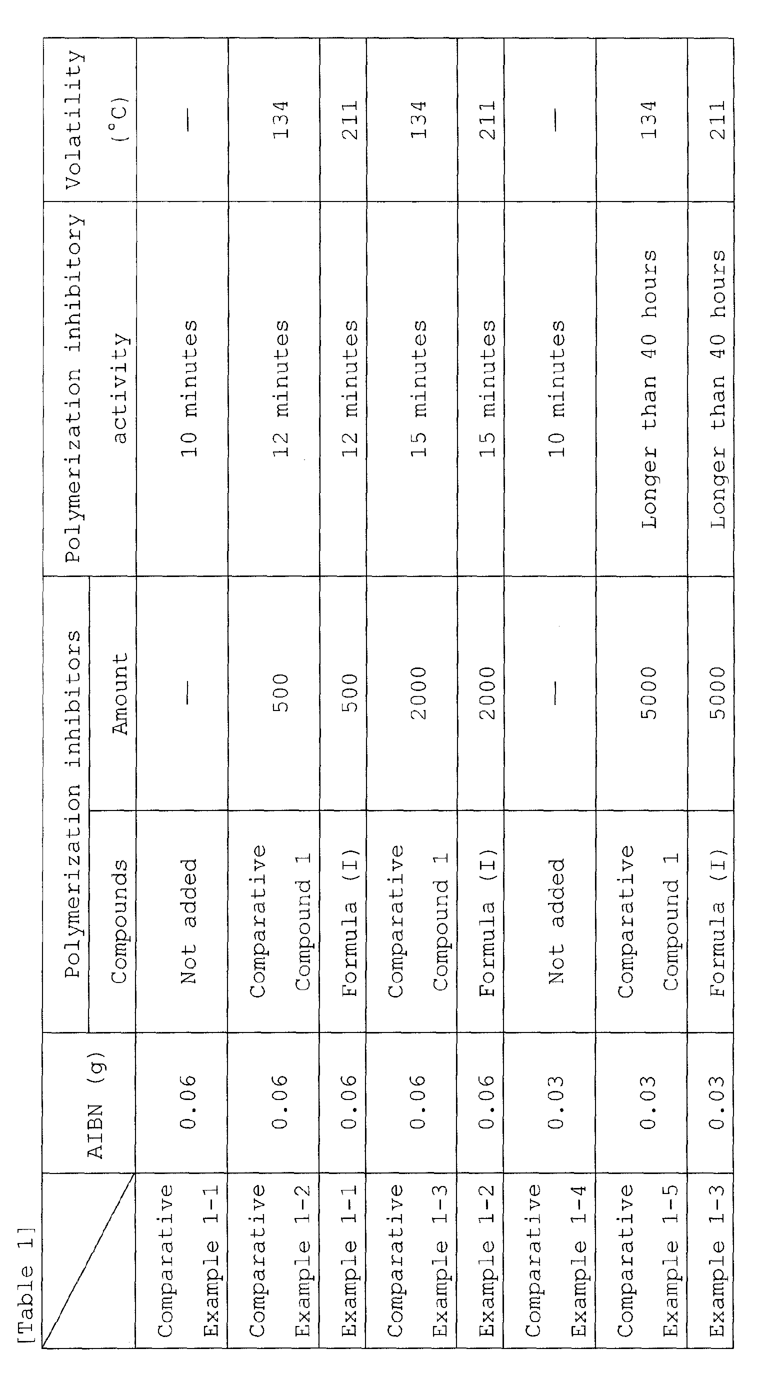

Table 1 confirms that the nitroxyl compound according to the present invention has polymerization inhibitory activity close to that of the known polymerization inhibitor and the 5% weight loss temperature of the nitroxyl compound is 77°C higher than that of the known polymerization inhibitor. This shows that the nitroxyl compound can function as a polymerization inhibitor which has excellent volatile resistance and which has high polymerization inhibitory activity.

### Example 2-1 and Comparative Examples 2-1 to 2-3

Comparative Compound 2 (bis(1-oxy-2,2,6,6-tetramethyl-4-piperidyl) sebacate) was synthesized as described below.

The following compounds were mixed together: 4 ml of 1-oxy-2,2,6,6-tetramethyl-4-hydroxypiperidine, 1 ml of methyl sebacate, 0.06 g of sodium methoxide, and 30 ml of heptane. Methanol was removed from the mixture under reflux. After the absence of methyl sebacate in the reaction liquid was confirmed by gas chromatography, the reaction liquid was washed with water such that sodium methoxide and 1-oxy-2,2,6,6-tetramethyl-4-hydroxypiperidine were removed from the reaction liquid. The solvent was removed from the reaction liquid washed with water, whereby a solid was obtained. The solid was recrystallized from hexane, whereby 1.5 g of bis(1-oxy-2,2,6,6-tetramethyl-4-piperidyl) sebacate was obtained.

In 25 ml of n-hexadecane, 10 mg of each polymerization inhibitor shown in Table 2 was dissolved, whereby a solution was prepared. A mixture of 1 µl of the solution and 5 µl of stearyl methacrylate was heated from room temperature to 150°C at 15°C/minute in a differential scanning calorimeter. The time from the moment of reaching 150°C to an exothermic peak due to polymerization was thereby measured and defined as polymerization inhibitory activity (hours). The measurement results are summarized in Table 2.

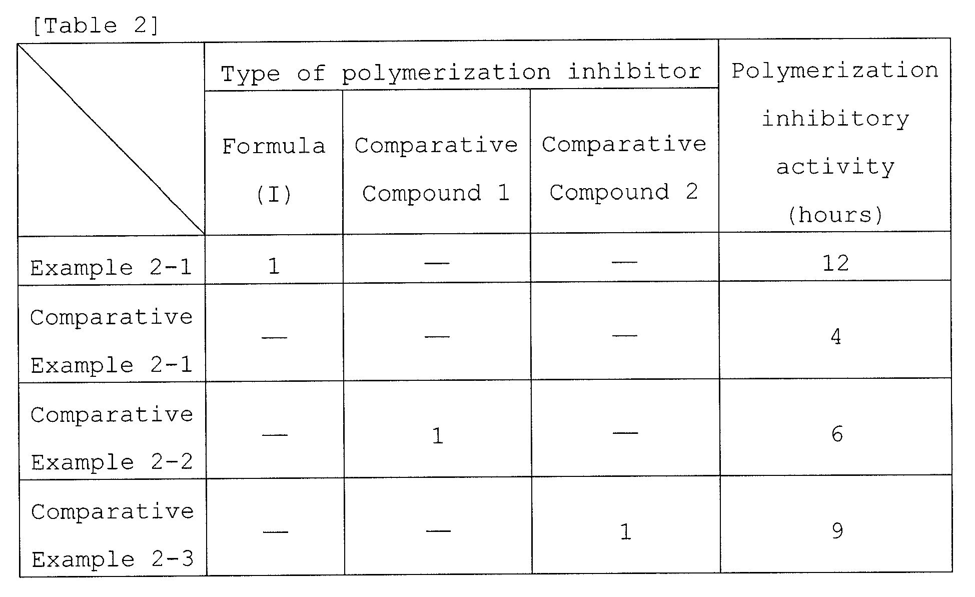

As is clear from Table 2, the compound according to the present invention is suitable for the long-term stabilization of a monomer and exhibits a stabilizing effect higher than that of Comparative Compound 1, which has a low molecular weight, and that of Comparative Compound 2, which has a high molecular weight. As a polymerization inhibitor, the compound according to the present invention is clearly superior to a known hindered amine compound having a nitroxyl structure.

## Claims

1. A nitroxyl compound represented by the following formula:

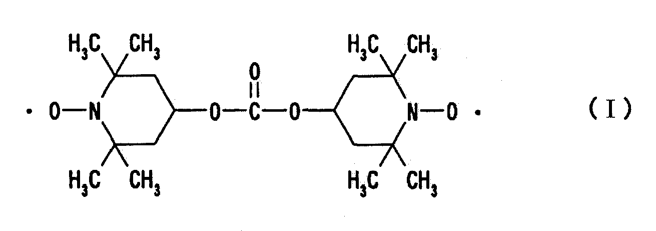

2. A polymerization inhibitor containing the nitroxyl compound according to Claim 1.

3. A polymerization inhibitor composition containing the nitroxyl compound according to Claim 1.
